# EUROPEAN PATENT APPLICATION

(11) **EP 2 842 601 A1**
(43) Date of publication of application: **04.03.2015**
(21) Application number: 12881527.1
(22) Date of filing: 15.02.2012
(51) Int. Cl.: A61N 5/02

(54) **MULTI-FREQUENCY MICROWAVE ACUPUNCTURE INSTRUMENT**

(30) Priority: 31.01.2012 CN 201210021439
(71) Applicant: Liu, Zhongyi, Gulou District Nanjing Jiangsu 210013 (CN)
(72) Inventor: Liu, Zhongyi, Gulou District Nanjing Jiangsu 210013 (CN)
(74) Representative: Kramer Barske Schmidtchen Patentanwälte PartG mbB
(86) International application number: PCT/CN2012/000180
(87) International publication number: WO 2013/120224

(57) **Abstract**

The present invention discloses an acupuncture and moxibustion instrument with multi-frequency microwave comprising a microwave oscillator A, a power amplifier A, a circulator A, a power divider A, a microwave radiator A, a microwave oscillator B, a power amplifier B, a circulator B, a power divider B, a microwave radiator B, a controller, a display screen, and a power supply unit. Advantages: Two medical frequencies in the international microwave band L are selected to avoid interference to communication and TV (oscillator A operates at 433MHz and oscillator B operates at 915MHz). The solid state modular microwave source used has good thermal penetration depth, heating homogeneity, and safety performance, as well as high efficiency and long service life. For both operating frequencies, continuous wave and 4 types of pulse modulated wave are available to treat different symptoms of illness. Dual effect of acupuncture using metal needle and heating of acupuncture points by moxibustion is provided. The treatment effect is good and the operation is easy. Good effect is provided for phlogosis, trigeminal neuralgia, and apoplexia and hemi-paralysis etc. One further advantage is absence of trauma, pain, and cross contamination.

## Description

### Technical field

This invention relates to an acupuncture and moxibustion instrument with multi-frequency microwave, in the field of medical appliance.

### Background of the invention

Existing technologies in this field have disadvantages. For example, in the patent of application No. of 95116466.x, the microwave source is a magnetron operating at 2450MHz, and the thermal penetration depth is only 1.5cm. The power consumption is high but the efficiency is low. In the patent of No.ZL93111800.x, although innovative solid state modular transmitter is used as medical appliance, limited by technical conditions at that time (1989), the microwave oscillator used is discrete component LC Clapp circuit, and for controller, timer, pulse modulator, and temperature measuring and control devices, analog circuits, mechanical switches, and nixie tubes were used.

### Summary of the invention

This invention discloses a type of acupuncture and moxibustion instrument with multi-frequency microwave, with the purpose to overcome aforesaid shortcomings of existing technology. This instrument adopts flush type microprocessor, touch type liquid crystal screen, subminiature high efficiency multi-purpose radiator, and solid state modular microwave source (transmitter), so that the subminiature high efficiency multi-purpose radiator (antenna) radiates microwave to relevant acupuncture points of patient, carried to the channels and collaterals, thus dredging the channels and collaterals, regulating qi and the blood, balancing yin yang, and strengthening the body resistance, and eliminating pathogenic factors, so as to effectively cure illness.

Technical solution of this invention: The acupuncture and moxibustion instrument with multi-frequency microwave of this invention comprises microwave oscillator A, power amplifier A, circulator A, power divider A, microwave radiator A, microwave oscillator B, power amplifier B, circulator B, power divider B, microwave radiator B, controller, display screen, and power supply unit, wherein said microwave oscillator A is connected in series with power amplifier A, circulator A, power divider A, and microwave radiator A; and said microwave oscillator B is connected in series with power amplifier B, circulator B, power divider B, and microwave radiator B; first signal output of said controller is connected to signal input of said microwave oscillator A, second signal output of said controller is connected to signal input of said power amplifier A, third signal output of said controller is connected to signal input of said microwave oscillator B, fourth signal output of said controller is connected to signal input of power amplifier B, and fifth signal output of said controller is connected to signal input of said display screen; power supply input of said controller is connected to power supply output of said power supply unit; and power supply input of said display screen is connected to power supply output of said power supply unit.

Advantages of this invention:
1) 2 medical frequencies in the international microwave band L are selected to avoid interference to communication and TV (oscillator A operates at 433MHz and oscillator B operates at 915MHz). Thermal penetration is deep and heating homogeneity is good. The solid state modular microwave source used has leakage performance far better than national standard, and good safety performance, high efficiency, and long service life.
2) This instrument has two operating frequencies. Continuous wave and 4 types of pulse modulated wave (1, 5, 10, 100Hz) can be selected to treat different symptoms of illness.
3) The subminiature high efficiency radiator is the key component of this instrument. To adapt to acupuncture points, minimum outline dimensions are: Φ25x8mm for radiator A (5) and Φ18x8mm for radiator B (10). Since operating frequencies are in microwave band L, the difficulty in R&D of subminiature high efficiency radiator is great. Success has only been gained by repeated testing and clinical tests.
4) This invention has dual effect of acupuncture using metal needle and heating of acupuncture points by moxibustion. Good effect is provided for phlogosis, trigeminal neuralgia, and apoplexia and hemi-paralysis etc. Further advantages include absence of trauma, pain, and cross contamination, and easy operation.
5) This instrument has been tried by specialists and professors in acupuncture and moxibustion of many hospitals. In their opinion, this instrument has good curative effect, easy operation, and good safety performance, and constitutes an innovation in the medicine of acupuncture and moxibustion.
6) Microwave oscillator used has been changed to voltage controlled oscillator (VCO), which has greatly improved frequency stability and accuracy.
7) Circuits of controller, pulse modulation, timer, and temperature measuring and control etc. adopt new flush type large scale integrated microprocessor, to provide greatly improved detection and control precision.
8) The 10.2-inch color touch type liquid crystal screen used can display various parameters, human body acupuncture point diagram, and acupuncture point formula recommended by WHO to various countries in a straightforward manner.

### Operating principle of this invention

The microwave source (transmitter) uses subminiature high efficiency radiator (antenna) to radiate microwave to relevant acupuncture points of patient, carried to the channels and collaterals, thus dredging the channels and collaterals, regulating qi and the blood, balancing yin yang, and strengthening the body resistance, and eliminating pathogenic factors, so as to effectively cure illness. In the opinion of Western medicine, the mechanism of microwave treatment is biological thermal effect and biochemical non-thermal effect, through anatomy analysis.

### Description of drawing figure

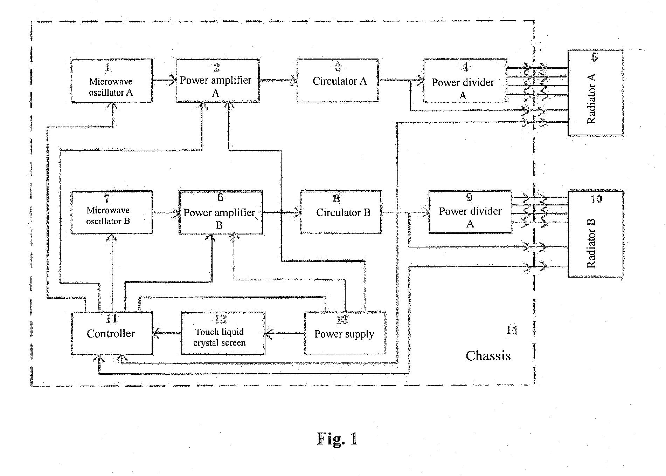

Fig.1: structural schematic block diagram of acupuncture and moxibustion instrument with multi-frequency microwave

In this figure, 1 is microwave oscillator A, 2 is power amplifier A, 3 is circulator A, 4 is power divider A, 5 is microwave radiator A, 6 is microwave oscillator B, 7 is power amplifier B, 8 is circulator B, 9 is power divider B, 10 is microwave radiator B, 11 is controller, 12 is display screen, 13 is power supply unit, and 14 is chassis.

### Preferred embodiment

As shown in the attached figure, the acupuncture and moxibustion instrument with multi-frequency microwave comprises of microwave oscillator A (1), power amplifier A (2), circulator A (3), power divider A (4), microwave radiator A (5), microwave oscillator B (6), power amplifier B (7), circulator B (8), power divider B (9), microwave radiator B (10), controller (11), display screen (12), and power supply unit (13), wherein said microwave oscillator A (1) is connected in series with power amplifier A (2), circulator A (3), power divider A (4), and microwave radiator A (5); and said microwave oscillator B (6) is connected in series with power amplifier B (7), circulator B (8), power divider B (9), and microwave radiator B (10); first signal output of said controller (11) is connected to signal input of said microwave oscillator A (1), second signal output of said controller (11) is connected to signal input of said power amplifier A (2), third signal output of said controller (11) is connected to signal input of said microwave oscillator B (6), fourth signal output of said controller (11) is connected to signal input of power amplifier B (7), and fifth signal output of said controller (11) is connected to signal input of said display screen (12); power supply input of said controller (11) is connected to power supply output of said power supply unit (13); and power supply input of said display screen (12) is connected to power supply output of said power supply unit (13).

Shape of microwave radiator A can be: 1) cylindrical (3 types): Φ25x8 mm, Φ31x8 mm, and Φ49x14 mm; 2) flat cylindrical: Φ67x26 mm; 3) flared: Φ100x14 mm; shape of microwave radiator B can be cylindrical (4 types): Φ18x8 mm, Φ25x8 mm, Φ49x14 mm, and Φ19x200 mm; and various combinations of such microwave radiator A and microwave radiator B are available to treat different symptoms of illness.

Microwave oscillator A (1) generates continuous wave and 1Hz/5Hz/10Hz/100Hz pulse modulated waves of operating frequency of 433MHz for output to power amplifier A (2), where they are amplified, and then output to circulator A (3). This circulator A (3) isolates these waves and sends them to power divider A (4), which divides microwave source power into 4 lines for output to the 4 radiators A (5).

Microwave oscillator B (6) generates continuous wave and 1Hz/5Hz/10Hz/100Hz pulse modulated waves of operating frequency of 915MHz for output to power amplifier B (7), where they are amplified, and then output to circulator B (8). This circulator B (8) isolates these waves and sends them to power divider B (9), which divides microwave source power into 4 lines for output to the 4 radiators B (10).

Microwave radiator A (5) and microwave radiator B (10) have various shapes for treatment of different symptoms of illness and multiple acupuncture points.

Operation process of this instrument is mainly realized by controller (model 9S08DZ60) 11.

### Functions of controller (11):

1) Control output waveform of microwave oscillator A (1) and microwave oscillator B (6). Output waveforms include continuous wave, 1Hz pulse modulated wave, 5Hz pulse modulated wave, 10Hz pulse modulated wave, and 100Hz pulse modulated wave. The pulse modulated waves are most suitable for acupuncture treatment of deep tissue diseases, e.g. prostatitis, periarthritis humeroscapularis, arthritis, and mastitis etc. Select a proper wave for treatment.
2) Set treatment period: When set period expires, an audible sound will be generated and the instrument will automatically stop.
3) Set control temperature: Treatment temperature is detected in real-time. In case of over-temperature, an alarm will be automatically generated and power output will be cut off.
4) Control and adjust microwave power output.
5) The control display screen (12) displays various settings, treatment parameters, human body acupuncture points diagram, and acupuncture point formula recommended by WHO.

Outline dimensions (mm) of microwave radiators: Microwave radiator A (1) has two shapes: 1) cylindrical: Φ25x8, Φ31x8, and Φ49x14; 2) flat cylindrical: Φ67x26, flared shape: Φ100xΦ25x14; total 6 types. Microwave radiator B (6) has four cylindrical shapes: Φ18x8, Φ25x8, Φ49x14, and Φ19x200; total 4 types. Microwave radiators A and B have a total of 10 types for treatment of different symptoms of illness.

### Instance of treatment: gonarthritis, with joint inflame, pain, and dropsy:

1. Select operating frequency A (433MHz); 2. Select 1Hz or 5Hz pulse modulated wave;
3. Select Φ25x8 radiator A to be placed at acupuncture points.
   Place one radiator at each hsiyen point. Place one radiator at the Yangguan point of the knee and one at Ququan point. Use adhesive plaster to fix them tight.
4. Turn on the instrument and adjust the power till comfortable warmth is felt. 5. Set treatment period to 30min.
6. Use this treatment once everyday. Normally, after treatment for 3∼5 times, the pain can be reduced, and subsidence of swelling will be gained. The curative effect is apparent.

## Claims

1. An acupuncture and moxibustion instrument with multi-frequency microwave, comprising a microwave oscillator A, a power amplifier A, a circulator A, a power divider A, a microwave radiator A, a microwave oscillator B, a power amplifier B, a circulator B, a power divider B, a microwave radiator B, a controller, a display screen, and a power supply unit, wherein said microwave oscillator A is connected in series with said power amplifier A, said circulator A, said power divider A, and said microwave radiator A; and said microwave oscillator B is connected in series with said power amplifier B, said circulator B, said power divider B, and said microwave radiator B; first signal output of said controller is connected to signal input of said microwave oscillator A, second signal output of said controller is connected to signal input of said power amplifier A, third signal output of said controller is connected to signal input of said microwave oscillator B, fourth signal output of said controller is connected to signal input of power amplifier B, and fifth signal output of said controller is connected to signal input of said display screen; power supply input of said controller is connected to power supply output of said power supply unit; and power supply input of said display screen is connected to power supply output of said power supply unit.

2. The acupuncture and moxibustion instrument with multi-frequency microwave according to claim 1, wherein shape of said microwave radiator A can be: 1) cylindrical (3 types): Φ25x8 mm, Φ31x8 mm, and Φ49x14 mm; 2) flat cylindrical: Φ67x26 mm; 3) flared: Φ100x14 mm; shape of said microwave radiator B can be cylindrical (4 types): Φ18x8 mm, Φ25x8 mm, Φ49x14 mm, and Φ19x200 mm; and various combinations of such microwave radiator A and microwave radiator B are available to treat different symptoms of illness.
